# EUROPEAN PATENT SPECIFICATION

(11) **EP 3 643 278 B1**
(45) Date of publication and mention of the grant of the patent: **08.09.2021**
(21) Application number: 18821401.9
(22) Date of filing: 22.05.2018
(51) Int. Cl.: A61F 13/47, A61F 13/533, A61F 13/536

(54) **ABSORBENT ARTICLE**
ABSORBIERENDER ARTIKEL
ARTICLE ABSORBANT

(30) Priority: 19.06.2017 JP 2017119742
(43) Date of publication of application: 29.04.2020
(73) Proprietor: Daio Paper Corporation, Ehime 799-0492 (JP)
(72) Inventor: TOKUNAGA, Sachiko, Sakura-shi Tochigi 329-1411 (JP)
(74) Representative: Epping - Hermann - Fischer
(86) International application number: PCT/JP2018/019688
(87) International publication number: WO 2018/235497

(56) References cited:
- EP-A1- 2 380 541
- EP-A1- 2 517 682
- WO-A1-2017/082056
- WO-A1-2018/003469
- JP-A- 2011 130 962

## Description

### Technical Field

The present invention relates to an absorbent article.

### Background Art

Conventionally, as absorbent articles such as a sanitary napkin, a panty liner, and an incontinence pad, a structure is known in which an absorbent body is disposed between a liquid-permeable topsheet, and a liquid-impermeable backsheet. Among such absorbent articles, an absorbent article having an increased fittability in a region starting from a part corresponding to a body fluid discharge opening to a rear part to prevent leaks of the body fluid from the rear part is known.

For example, Patent Literature 1 discloses an absorbent article in which each of side regions on both sides of an intermediate central region has lower stiffness than side regions of a front central region and a rear central region.

Patent Literature 2 discloses a structure including arc-like embosses corresponding to deformation of buttocks formed on both sides of a longitudinal central line, each arc having a center of curvature outside of the absorbent article in a width direction; and an emboss corresponding to an intergluteal cleft having an inverted V-shaped portion, the inverted V-shape starting from the position on the longitudinal central line rearwardly gradually diverging to both sides.

Patent Literature 3 relates to an absorptive article such as a sanitary napkin, a pantiliner, an incontinence pad and the like for absorbing menstrual blood, vaginal discharge and the like, or more particularly to an absorptive article which fits the shape and movement of a body, eliminates wrinkles and improves a wearing feeling.

Patent Literature 4 relates to an absorbent article used for sanitary napkins, pantiliners, incontinence pads and the like, which is enhanced in comfort and absorptivity thanks to deformation of the absorbent article during wearing.

### Citation List

### Patent Literature

Patent Literature 1: Japanese Patent No. 4323786
Patent Literature 2: Japanese Laid-open Patent Publication No. 2011-156070
Patent Literature 3: EP 2 517 682 A1
Patent Literature 4: EP 2 380 541 A1

### Summary of Invention

### Technical Problem

In Patent Literature 1, it is described that the disclosed structure enables the front part and the rear part of the absorbent article to move individually following the movement of a body. Further, in Patent Literature 2, it is described that the absorbent article can be fitted corresponding to deformation of buttocks during sleeping time.

However, in the absorbent articles disclosed in Patent Literatures 1 and 2, a scope for improvement is associated with a more appropriate deformation to adapt to the shape of the intergluteal cleft.

In light of the above, an object of the present invention is to provide an absorbent article which is well deformable following the shape of the intergluteal cleft, and thereby preventing leakage in the rear region of the absorbent article.

### Solution to Problem

A first embodiment of the present invention provides an absorbent article including a main body including a liquid-permeable topsheet, a liquid-impermeable backsheet, and an absorbent body disposed between the topsheet and the backsheet, wherein the main body has an elongated shape with a predetermined length in the front-rear direction and a predetermined width in a width direction orthogonal to the front-rear direction, and includes a compressed groove recessed from a topsheet side toward a backsheet side, wherein the compressed groove includes a pair of front-rear direction compressed grooves extending in the front-rear direction, wherein the front-rear direction compressed grooves each include high-compressed portions and a low-compressed portion that is less deeply recessed than the high-compressed portions, and each include a first rear compressed groove formed in a region corresponding to an intergluteal cleft of a wearer when attached, and a middle compressed groove formed forward of the first rear compressed groove, and wherein a total area per unit length in the front-rear direction of the high-compressed portions in the first rear compressed groove is smaller than a total area per unit length in the front-rear direction of the high-compressed portions in the middle compressed groove.

### Advantageous Effects of Invention

According to one embodiment of the present invention, there is provided an absorbent article which is well deformable following the shape of the intergluteal cleft, thereby preventing leakage in the rear part.

In the embodiment, the front-rear direction compressed groove includes the first rear compressed groove formed in the region corresponding to an intergluteal cleft of the wearer when attached, and the middle compressed groove formed forward of the first rear compressed groove. Further, the total area per unit length in the front-rear direction of the high-compressed portions in the first rear compressed groove is smaller than the total area per unit length in the front-rear direction of the high-compressed portions in the middle compressed groove. Therefore, the rigidity of the region corresponding to the intergluteal cleft (the intergluteal cleft corresponding region), in which the first rear compressed groove is formed, can be lower than the rigidity of the middle region of the main body, in which the middle compressed groove is formed. This allows for maintaining the softness of the main body in the region corresponding to the intergluteal cleft, and for deforming the main body well following the shape of the intergluteal cleft. As a result, the absorbent article can be well fitted to the intergluteal cleft, with no gap between buttocks and the main body, thereby preventing rearward leakage of body fluid.

Preferred embodiments of the present invention are described below as appendices.

### (Embodiment 2)

Embodiment 2 provides that a groove width of the first rear compressed groove is smaller than a groove width of the middle compressed groove.

In the Embodiment 2, the decreased width of the rear compressed groove can lower the rigidity attributed to the first rear compressed groove. Therefore, the rigidity of the main body in the intergluteal cleft corresponding region can be lowered, enhancing the effect that the main body can be well deformed following the shape of the intergluteal cleft.

### (Embodiment 3)

Embodiment 3 provides that a pitch of the high-compressed portions in the first rear compressed groove is greater than a pitch of the high-compressed portions in the middle compressed groove.

In the Embodiment 3, the greater pitch of the high-compressed portions in the first rear compressed groove can lower the rigidity attributed to the first rear compressed groove. Therefore, the rigidity of the main body in the intergluteal cleft corresponding region can be lowered, enhancing the effect that the main body can be well deformed following the shape of the intergluteal cleft.

### (Embodiment 4)

Embodiment 4 provides that the front-rear direction compressed grooves each include a second rear compressed groove formed backward of the first rear compressed groove, and a total area per unit length in the front-rear direction of the high-compressed portions in the second rear compressed groove is greater than the total area per unit length in the front-rear direction of the high-compressed portions in the first rear compressed groove.

In the Embodiment 4, the front-rear direction compressed grooves each include the second rear compressed groove rearward of the first rear compressed groove, and the total area of the high-compressed portions in the second rear compressed groove is greater than the total area of the high-compressed portions in the first rear compressed groove, per unit length in the front-rear direction. With this configuration, the rigidity of a further rear region rearward of the intergluteal cleft corresponding region, i.e., the region in which the second rear compressed groove is provided, can be higher than the rigidity of the intergluteal cleft corresponding region. Therefore, twists and wrinkles can be prevented in the region closer to the rear end, while maintaining the effect that the rigidity in the intergluteal cleft corresponding region can be lowered to maintain the softness of the main body, and thus the main body can be well deformed following the shape of the intergluteal cleft.

### (Embodiment 5)

Embodiment 5 provides that a groove width of the second rear compressed groove is greater than a groove width of the first rear compressed groove.

In the Embodiment 5, the greater groove width of the second rear compressed groove can enhance the rigidity of the region rearward of the intergluteal cleft corresponding region, closer to the rear end. Therefore, twists and wrinkles in the region rearward of the intergluteal cleft corresponding region can be prevented, while maintaining the effect that the main body can be well deformed following the shape of the intergluteal cleft in the intergluteal cleft corresponding region.

### (Embodiment 6)

Embodiment 6 provides that a pitch of the high-compressed portions in the second rear compressed groove is smaller than the pitch of the high-compressed portions in the first rear compressed groove.

In the Embodiment 6, the reduced pitch in the second rear compressed groove can increase the rigidity in the region rearward of the intergluteal cleft corresponding region, closer to the rear end. Therefore, twists and wrinkles in the region rearward of the intergluteal cleft corresponding region can be prevented, while maintaining the effect that the main body can be well deformed in the intergluteal cleft corresponding region following the shape of the intergluteal cleft.

### Brief Description of Drawings

FIG. 1 is a partially cutaway view showing an absorbent article according to one embodiment of the present invention.
FIG. 2 is a cross-sectional view taken along the line IV-IV in FIG. 1.
FIG. 3A is a view illustrating deformation of an intergluteal cleft corresponding region in an absorbent article according to an embodiment of the present invention when attached.
FIG. 3B is a view illustrating deformation of an intergluteal cleft corresponding region in an absorbent article according to the prior art when attached.
FIG. 4A is an enlarged view showing a middle compressed groove in an embodiment of the present invention.
FIG. 4B is an enlarged view showing a first rear compressed groove in an embodiment of the present invention.
FIG. 4C is an enlarged view showing a second rear compressed groove in an embodiment of the present invention.
FIG. 5 is a view of an absorbent article according to another embodiment of the present invention.

### Description of Embodiments

An embodiment of the present invention will now be described with reference to the drawings.

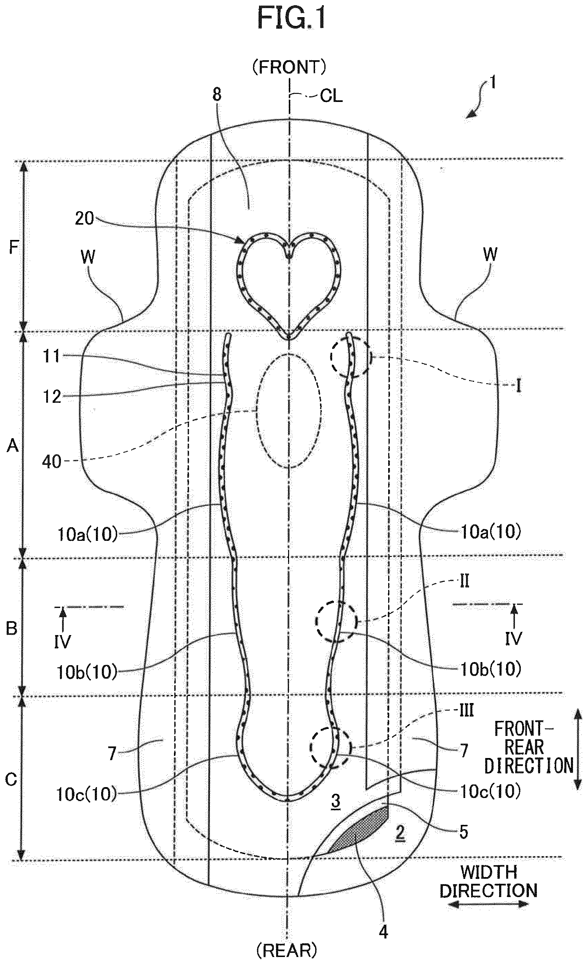

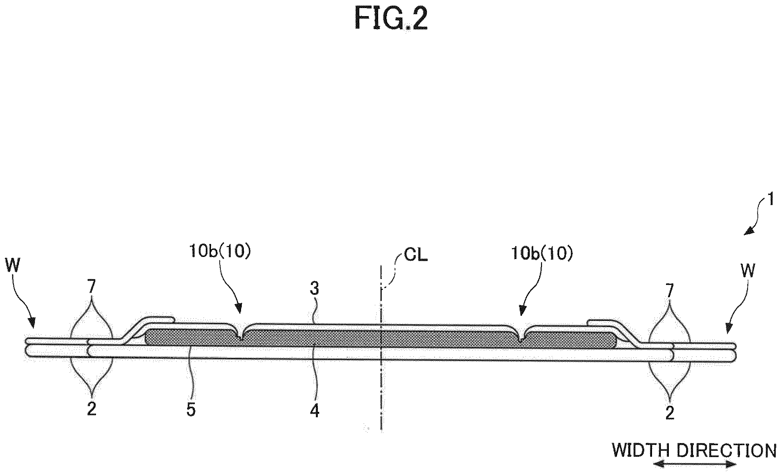

FIG. 1 is a partially cutaway view of an absorbent article 1 according to an embodiment of the present invention. FIG. 2 is a cross-sectional view taken along IV-IV line in FIG. 1. As shown in FIGs. 1 and 2, the absorbent article 1 includes a main body (absorbent article main body) 8 including a liquid-impermeable backsheet 2, a liquid-permeable topsheet 3, and an absorbent body 4 disposed between the backsheet 2 and the topsheet 3. The absorbent body 4 may be wrapped by an enveloping sheet made of crepe paper, nonwoven fabric, or the like, for the purpose of maintaining a shape of the absorbent body 4, etc.

As shown in FIG. 1, the main body 8 has an elongated shape in its entirety with a predetermined length in a front-rear direction and a substantially constant width in a direction orthogonal to the front-rear direction. In the illustrated embodiment, the absorbent article 1 has a substantially line-symmetric shape with respect to a centerline CL extending in the front-rear direction. However, the structure (including the shape) of the absorbent article 1 or the main body 8 is not necessarily line-symmetric. Further, the absorbent article 1 may have wings W, W to ensure a reliable fixation of the absorbent article 1 to underwear when attached. In the illustrated example, the wings W, W extend from each side of a region of the main body 8 including a site corresponding to a body fluid discharge opening (body fluid discharge opening corresponding portion) 40. However, the absorbent article 1 may not necessarily have wings W, W.

At the front and rear end portions of the absorbent body 4, the outer edges of the backsheet 2 and the topsheet 3 are bonded to each other by an adhesive such as a hot-melt adhesive, or by bonding means such as a heat seal, and an ultrasonic seal. Further, a side nonwoven fabric 7 is arranged along the front-rear direction (longitudinal direction) on each of the lateral parts on the topsheet side. The side nonwoven fabric 7 is partially protruded laterally from the main body 8, and laminated to the backsheet 2, which is also protruded laterally from the main body 8, by an adhesive such as a hot-melt adhesive, or bonding means such as a heat seal, and an ultrasonic seal, thereby forming the wings W, W on the both lateral sides of the main body 8.

The backsheet 2 may be made of a sheet material having at least water-blocking properties. For example, an olefin resin sheet such as polyethylene or polypropylene may be used. A laminated nonwoven fabric, which is formed by laminating a nonwoven fabric on a polyethylene sheet or the like, or a laminated sheet of nonwoven fabric layers, which has a waterproof film interposed therein to secure substantial liquid-impermeability may be used. Further, a moisture-permeable sheet is preferably used in order to prevent stuffiness. An example of such water-impermeable/moisture-permeable sheet material includes a microporous sheet formed by melt-kneading an olefin resin such as polyethylene or polypropylene with an inorganic filler to form a sheet, and then stretching the sheet in a uniaxial direction or biaxial directions.

The topsheet 3 is a liquid-permeable sheet that allows quick passage of body fluid such as menstrual blood, vaginal discharge, or urine. As the topsheet 3, a porous or nonporous nonwoven fabric or a porous plastic sheet is preferably used. Examples of fiber materials for the nonwoven fabric include synthetic fibers of an olefin such as polyethylene or polypropylene, a polyester, a polyamide, or the like; regenerated fibers such as rayon and cupra; blended fibers of these; and natural fibers such as cotton. These fibers may be used alone or in any combination. Further, methods of processing the nonwoven fabric may include spunlacing, spunbonding, thermal bonding, melt blowing, and needle punching. Among these processing methods, the spunlacing is preferable in being capable of manufacturing a nonwoven fabric with good flexibility, the spunbonding is preferable in being capable of manufacturing a nonwoven fabric with good drapability, and the thermal bonding is preferable in being capable of manufacturing a bulky, soft nonwoven fabric. Further, composite fibers such as sheath-core fibers having a high melting point fiber as a core and a low melting point fiber as a sheath, side-by-side fibers, and split fibers may be used.

The material for the absorbent body 4 interposed between the backsheet 2 and the topsheet 3 is not particularly limited as long as being capable of absorbing and retaining body fluids, but for example, the absorbent member preferably includes cotton-like pulp and a water absorptive polymer. Examples of the water absorptive polymer may include superabsorbent polymer (SAP), superabsorbent fiber (SAF), and combinations thereof. Examples of pulp include cellulose fibers such as chemical pulp obtained from wood, dissolving pulp, and the like, and also artificial cellulose fibers such as rayon, and acetate. Hardwood materials, softwood materials, and the like may be used as the raw material for the chemical pulp, but the softwood materials are preferably used due to their long fiber length, etc.

A synthetic fiber may be mixed in the absorbent body 4. Examples of the synthetic fiber may include polyolefins such as polyethylene and polypropylene, polyesters such as polyethylene terephthalate, and polybutylene terephthalate, polyamides such as nylon, and copolymers thereof. Two or more of these materials may also be used in mixture. Also, multi-component fibers such as sheath-core fibers having a core fiber with a high melting point and a sheath fiber with a low melting point, side-by-side fibers, split fibers, and the like may also be used. Hydrophobic fiber subjected to a surface treatment with a hydrophilizing agent to exhibit affinity to body fluid may be used.

The thickness of the absorbent body 4 is within the range from 0.5 mm to 25 mm, preferably within the range from 3.5 mm to 7.5 mm. The absorbent body 4 may not necessarily have a uniform thickness across its entire surface. For example, the absorbent body 4 may have a bulging in the body fluid discharge opening corresponding portion 40 and the vicinity thereof, or in the portion corresponding to the intergluteal cleft (intergluteal cleft corresponding portion) and the vicinity thereof. Further, the absorbent body 4 is preferably manufactured by a stacking process or an air laid process.

Examples of the material that may be used as the side nonwoven fabric 7 include a water-repellent treated nonwoven fabric, and a hydrophilically treated nonwoven fabric. For example, for enhancing the anti-permeation effect against menstrual blood, vaginal discharge, or the like, or improving the feel of texture, a water-repellent treated nonwoven fabric coated with a silicone, paraffin, or alkyl chromic chloride water repellent, or the like may be used. For enhancing absorbability of menstrual blood or the like at the wing W, a hydrophilically treated nonwoven fabric may be used as the material of the nonwoven fabric. A preferable type of the nonwoven fabric is an air-through nonwoven fabric that is less likely to develop folds, wrinkle-resistant, and soft.

On the outer edge of the wings W, W, for the purpose of bonding the side nonwoven fabric 7 and the backsheet 2, and increasing rigidity, dotted compressed portions (embosses) or a compressed portion with a predetermined shape may be provided in a predetermined area.

The total length of the absorbent article 1 may be 130 to 450 mm, and also may be 200 to 360 mm.

The absorbent article 1 includes a compressed groove (also referred to as pressed groove, or emboss). The compressed groove is formed so as to be a linear groove recessed from the topsheet 3 side to the backsheet 2 side. The compressed groove functions to prevent the absorbent body 4 from being twisted, to control body fluid migration on the surface or in the inside of the absorbent body, and to facilitate an appropriate deforming following the shape of the body. The compressed groove may be formed by passing a stack including the absorbent body 4 and the topsheet 3 thereon between a pair of pressing rolls. For example, the stack may be let through so that a protruded roll is arranged on the topsheet 3 side and a planar roll is arranged on the absorbent body 4 side.

As shown in FIG. 1, the compressed groove includes a pair of front-rear direction grooves 10, 10 extending in the front-rear direction, line-symmetrically with respect to a front-rear direction centerline CL of the main body. The pair of front-rear direction compressed grooves 10, 10 may extend in the front-rear direction, along the lateral sides of the body fluid discharge opening corresponding portion 40, or through a position laterally away from the body fluid discharge opening corresponding portion 40 by a predetermined distance, so that the grooves 10, 10 are not on the portion corresponding to the body fluid discharge opening such as the vaginal opening when attached (the body fluid discharge opening corresponding portion) 40. The length of the front-rear direction compressed grooves 10, 10 may be approximately 50 to 85 % with respect to the length of the main body. Further, the front-rear direction compressed grooves 10, 10 may extend linearly as a whole, or may be curved in mid-course as shown in the illustrated example. A pair of front-rear direction grooves 10, 10 is not necessarily line-symmetric. The paired front-rear direction grooves 10, 10 are preferably respectively provided on each side of the front-rear direction centerline CL.

The pair of front-rear direction compressed grooves 10, 10 may function as base points (flexible shafts) at which the absorbent article main body 8 can be easily bent. Therefore, when forces of both legs are applied from lateral sides (in the direction orthogonal to front-rear direction) toward the main body 8, the front-rear direction compressed grooves 10, 10 can become base points for raising the region between the front-rear direction compressed grooves 10, 10. Accordingly, the fittability of the main body 8 to the human body can be enhanced.

A pair of front-rear direction compressed grooves 10, 10 includes high-compressed portions 11, and a low-compressed portion 12 that is less deeply depressed than in a high-compressed portions 11 (FIGs. 1 and 2). In the drawings, the high-compressed portions 11 are illustrated in black, and the low-compressed portion 12 is illustrated in white (with no coloring). The compression depths of the high-compressed portions 11 and the low-compressed portion 12 are not particularly limited, but the high-compressed portions 11 are formed to have a depth approximately 0.5 mm to 3 mm deeper than the bottom surface of the low-compressed portion 12. Further, in the front-rear direction compressed grooves 10, 10, the high-compressed portions 11 may be continuously or discontinuously formed in the front-rear direction. Preferably, the high-compressed portions 11 are discontinuously formed in the front-rear direction (the plurality of the high-compressed portions 11 are separated with each other in the front-rear direction), so that the grooves have an appropriate rigidity. Such structure of the compressed groove including the high-compressed portions and the low-compressed portion may be obtained by using pressure rolls having recesses and protrusions corresponding to a desired structure. For example, the pressure roll may be configured to have projections corresponding to the entire shape of the compressed grooves, and fine protrusions corresponding to the shape of the high-compressed portions on the projections. Alternatively, the low-compressed portions may be firstly formed at a relatively low pressure in the region where the compressed grooves are to be formed, and then the high-compressed portions may be formed partially in the region of the low-compressed portions. The depth of the compressed portions may be controlled by adjusting the pressure of the above-mentioned pressure rolls used for forming the compressed grooves.

In the present embodiment, the high-compressed portions and the low-compressed portion may be formed also in the compressed grooves other than the front-rear direction compressed grooves 10, 10. In the example illustrated in FIG. 1, the high-compressed portions and the low-compressed portion are formed in all compressed grooves including later-described front compressed groove 20.

In the example illustrated in FIG. 1, a pair of the front-rear direction compressed grooves 10, 10 includes middle compressed grooves 10a, 10a, first rear compressed grooves 10b, 10b, and second rear compressed grooves 10c, 10c, which may be connected in the stated order in the front-rear direction. The middle compressed grooves 10a, 10a are formed in the middle region A, the first rear compressed grooves 10b, 10b are formed in the first rear region B, and the second rear compressed grooves 10c, 10c are formed in the second rear region C.

The length in the front-rear direction of the middle compressed grooves 10a, 10a may be 60 to 110 mm, the length in the front-rear direction of the first rear compressed grooves 10b, 10b may be 30 to 60 mm, and the length in the front-rear direction of the second rear compressed grooves 10c, 10c may be 30 to 60 mm.

The middle region A includes the body fluid discharge opening corresponding portion 40, and may substantially correspond to a part from the urethral opening to the anus between the legs of the wearer when attached. Further, the first rear region B is the region corresponding to the intergluteal cleft of the wearer, more specifically, the region extending in the front-rear direction and including a part corresponding to the wearer's intergluteal cleft (intergluteal cleft corresponding region). The first rear region B may be a region covering the whole gluteal cleft, or a region corresponding to a part from a rear part of the perineum or the vicinity thereof, or from anus or the vicinity thereof to the lower end of tailbone or the vicinity. The second rear region C may substantially be a region from a position corresponding to a rear end of gluteal cleft to the rear end of the main body 8. The region forward of the middle region A may be a front region F.

As described above, when the main body 8 receives a force from the legs while being attached, the front-rear direction compressed grooves 10, 10 usually become base edges so that the region between the front-rear direction compressed grooves 10, 10 of the main body 8 can be highly raised. The highly raised region between the front-rear direction compressed grooves 10, 10 may usually be well fitted to a relatively narrow deep groove in crotch.

However, the intergluteal cleft is wider and less deep than the cleft in the crotch. Accordingly, in a case that the region between the front-rear direction compressed grooves 10, 10 is sharply raised, the main body 8 may not be well tightly fitted to the intergluteal cleft.

In light of the above, according to an example of the present invention, the pair of the front-rear direction compressed grooves 10, 10 includes the first rear compressed grooves 10b, 10b formed in a region B corresponding to the intergluteal cleft of the wearer when attached, and the middle compressed grooves 10a, 10a formed forward of the first rear compressed grooves 10b, 10b. Further, a total area per unit length Sb of all the high-compressed portions 11 of the first rear compressed grooves 10b, 10b in the front-rear direction is smaller than a total area per unit length Sa of the high-compressed portions 11 of the middle compressed groove 10a, 10a in the front-rear direction (Sb < Sa).

In the present specification, the "total area per unit length in the front-rear direction of the high-compressed portions" means a total area of all the high-compressed portions 11 within a region between two imaginary lines extending across the main body 8 in the width direction at a predetermined distance with each other. For example, the total area of all the high-compressed portions included within a region between two imaginary lines at a distance of 3 cm in the front-rear direction from each other may be calculated for each of the compressed grooves (for the middle compressed grooves 10a, 10a, and the first rear compressed grooves 10b, 10b), and each calculated total area may be compared. Further, the total areas calculated either on the left half side or the right half side of the front-rear direction centerline CL may be compared.

As the high-compressed portions 11 are portions intensively and deeply compressed by pressure rolls as described above, the smaller the area of the high-compressed portions 11 per unit length is, the less rigid the compressed groove becomes. Therefore, the rigidity of the first rear compressed grooves 10b, 10b is lower than the rigidity of the middle compressed grooves 10a, 10a, and thus the surrounding area of the first rear compressed grooves 10b, 10b is also less rigid. With this configuration, the first rear region B can be kept soft, allowing the main body 8 not to bend sharply at the first rear compressed grooves 10b, 10b as base points . Accordingly, the main body 8 can be well deformed following the shape of the intergluteal cleft, which is wider and less deep than the cleft in the crotch.

The ratio of the total area Sb per unit length in the front-rear direction of the high-compressed portions 11 in the first rear compressed grooves 10b, 10b to the total area Sa per unit length in the front-rear direction of the high-compressed portions 11 in the middle compressed grooves 10a, 10a (Sb/Sa) may be preferably 0.2 to 0.6, more preferably 0.32 to 0.52.

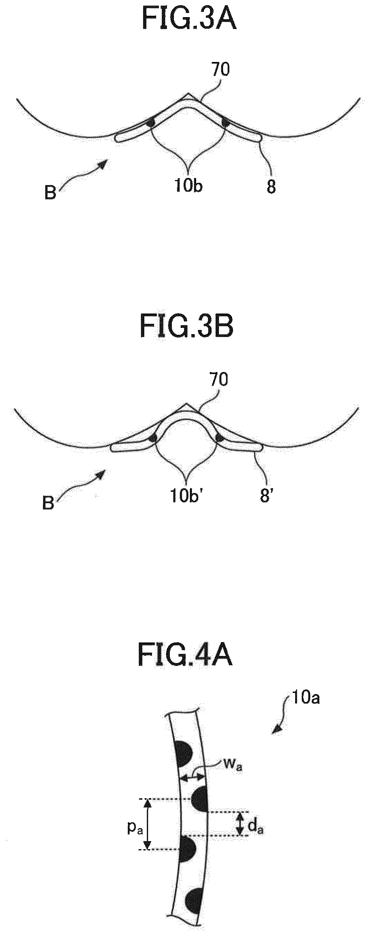

The function and effect of the structure of the compressed grooves according to the present example will be further described referring to FIG. 3A and 3B. FIG. 3A is a cross-sectional view showing how the main body 8 is contacted with the buttocks 70 when the absorbent article 1 in Fig. 1 is attached. On the other hand, FIG. 3B is a cross-sectional view showing how the main body 8' according to the prior art is contacted with buttocks cleft 70.

In FIG. 3B, the compressed grooves 10b', 10b' formed in the first rear region B of the conventional absorbent article main body 8' has the same structure and the same rigidity as the middle compressed grooves formed forward of the compressed grooves 10b', 10b'. Therefore, the main body 8' can be easily sharply bent at the compressed grooves 10b', 10b' as base points, and the region between the compressed grooves 10b', 10b' may likely be protruded and highly raised. Accordingly, the main body 8' cannot be well fitted to the shape of the wider cleft 70 of the buttocks, and a gap is likely formed between the buttocks and the main body 8', which may result in leakage of body fluid.

In the present example, the first rear compressed grooves 10b, 10b provided in the first rear region B of the absorbent article main body 8 have a different structure from the middle compressed grooves 10a, 10a formed forward of the first rear compressed grooves 10b, 10b (FIG. 1). Specifically, the total area Sb per unit length in the front-rear direction of the high-compressed portions 11 in the first rear compressed grooves 10b, 10b is lower than the total area Sa per unit length in the front-rear direction of the high-compressed portions 11 in the middle compressed grooves 10a, 10a, and thus the rigidity of the first rear compressed grooves 10b, 10b is smaller than the rigidity of the middle compressed grooves 10a, 10a. Therefore, the first rear compressed grooves 10b, 10b are not bent sharply, and the softness of the main body 8 as a whole can be maintained. The main body 8 is gently raised as shown in FIG. 3A, and is fitted along the shape of the intergluteal cleft 70. Accordingly, the leakage of the body fluid, especially the rearward leakage along the intergluteal cleft 70 can be prevented.

A distance between the first rear compressed grooves 10b, 10b is smaller than a distance between the middle compressed grooves 10a, 10a. With this configuration, the height of the raised region of the main body 8 in the first rear region B can be lower than in the middle region A, so that the first rear region B can be well fitted to the buttocks.

Further, since the softness of the main body 8 can be maintained in the region B corresponding to the intergluteal cleft as described above, the main body 8 can be deformed following the change of the width or the depth of the intergluteal cleft when the position of the human body is changed. Moreover, the middle compressed grooves 10a, 10a are connected to the first rear compressed grooves 10b, 10b, resulting in a good fittability to a transition part from the crotch part to the intergluteal cleft of the wearer. Therefore, the absorbent article 1 according to the present example can well prevent the rearward leakage even for a long time or during a sleeping time, and thus is preferably used as a sanitary napkin for long time-use or for sleeping time-use.

In the example shown in FIG. 1, as described above, a pair of the front-rear direction compressed grooves 10, 10 includes the middle compressed grooves 10a, 10a, the first rear compressed grooves 10b, 10b, and additionally the second rear compressed grooves 10c, 10c. A total area Sc per unit length in the front-rear direction of high-compressed portions in the second rear compressed grooves 10c, 10c may be greater than the total area Sb per unit length in the front-rear direction of the high-compressed portions in the first rear compressed grooves 10b, 10b (Sc > Sb). Therefore, the rigidity of the second rear compressed grooves 10c, 10c, which is closer to the rear end, can be higher than in the first rear compressed grooves 10b, 10b. Accordingly, twists and wrinkles are prevented in the second rear region C, which faces the part in which only a narrow or no intergluteal cleft exists, while maintaining the above-described effect that the rigidity in the first rear region (intergluteal cleft corresponding region) B can be lowered to maintain the softness of the main body, and the main body can be well deformed following the shape of the intergluteal cleft.

The ratio of the total area Sc per unit length in the front-rear direction of the high-compressed portions in the second rear compressed grooves 10c, 10c to the total area Sb per unit length in the front-rear direction of the high-compressed portions in the first rear compressed grooves 10b, 10b (Sc/Sb) is preferably 1 to 4.5, more preferably 1.5 to 3.5.

The total area Sc per unit length in the front-rear direction of the high-compressed portions in the second rear compressed grooves 10c, 10c may be the same as or different from the total area Sa per unit length in the front-rear direction of the high-compressed portions in the middle compressed grooves 10a, 10a.

In the illustrated example, the second rear compressed grooves 10c, 10c are more curved inwardly and have a shorter distance between the compressed grooves in the rear direction, to be connected with each other at the rear end. However, the shape in planar view of the second rear compressed grooves 10c, 10c is not limited to the illustrated example, and the second rear compressed grooves 10c, 10c may not necessarily be connected with each other.

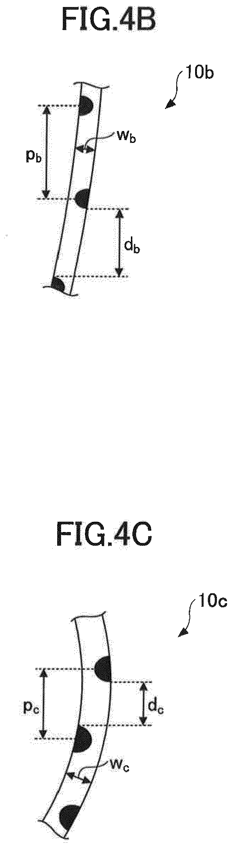

FIGs. 4A to 4C are enlarged views of part I, II, and III in FIG. 1, i.e., partially enlarged views of the middle compressed grooves 10a, 10a, the first rear compressed grooves 10b, 10b, and the second rear compressed grooves 10c, 10c, respectively. As illustrated, each of the compressed grooves has the high-compressed portions 11 and the low-compressed portion 12.

Further, as shown in FIG. 4A and FIG. 4B, a groove width w_{b} of the first rear compressed groove 10b is preferably less than a groove width wₐ of the middle compressed groove 10a (w_{b} < wₐ). Therefore, the rigidity of the first rear compressed groove 10b can be further lowered, thereby enhancing the function and the effect that the softness of the main body 8 can be maintained in the first rear region B.

The groove width w_{b} of the first rear compressed groove 10b is preferably 0.5 to 3.0 mm, and more preferably 1 to 2 mm. The groove width wₐ of the middle compressed groove 10a is preferably 1.0 to 3.5 mm, and more preferably 1.5 to 2.5 mm. The ratio of the w_{b} to the wₐ (w_{b}/wₐ) is preferably 0.60 to 0.9, and more preferably 0.65 to 0.85.

Further, as illustrated, a pitch p_{b} of the high-compressed portions 11 in the first rear compressed groove 10b is preferably greater than a pitch pₐ of high-compressed portions 11 in the middle compressed groove 10a (p_{b} > pₐ). Therefore, the area of the low-compressed portion 12 can be decreased in the first rear compressed groove 10b, thereby enhancing the function and effect that the rigidity of the first rear region B can be lowered and the softness of the main body 8 can be maintained in the first rear region B.

The pitch p_{b} of the high-compressed portions 11 in the first rear compressed groove 10b is preferably 4 to 10 mm, and more preferably 6 to 8 mm. The pitch pₐ of the high-compressed portions 11 in the middle compressed groove 10a is preferably 2.5 to 6 mm, and more preferably 3.4 to 4.4 mm. The ratio of the p_{b} to the pₐ (p_{b}/pₐ) is preferably 1.2 to 2.8, and more preferably 1.3 to 2.3.

A distance d_{b} between the high-compressed portions 11 in the first rear compressed groove 10b is also preferably greater than a distance dₐ between the high-compressed portions 11 in the middle compressed groove 10a (d_{b} > dₐ). The distance d_{b} between the high-compressed portions 11 in the first rear compressed groove 10b is preferably 2.5 to 7 mm, more preferably 3.5 to 6.5 mm. Further, the distance dₐ between the high-compressed portions 11 in the middle compressed groove 10a is preferably 0.5 to 3 mm, more preferably 1.2 to 2.3 mm.

As illustrated, a groove width w_{c} of the second rear compressed groove 10c is preferably greater than a groove width w_{b} of the first rear compressed groove 10b (w_{c} > w_{b}). This allows the rigidity of the second rear region C to be greater than the rigidity of the first rear region B. Therefore, the above-described effect to lower the rigidity of the first rear region B and to maintain the softness of the main body 8, enabling a deformation of the main body well following the shape of the intergluteal cleft, is achieved, and twists and wrinkles in the second rear region C closer to the rear end can be prevented.

A groove width w_{c} of the second rear compressed groove 10c is preferably 1.0 to 3.5 mm, more preferably 1.5 to 2.5 mm. Further, the ratio of the w_{c} to the w_{b} (w_{c}/w_{b}) is preferably 1.1 to 1.5, more preferably 1.2 to 1.4. The groove width w_{c} of the second rear compressed groove 10c may be the same as the groove width wₐ of the middle compressed groove 10a.

Further, a pitch p_{c} of the high-compressed portions 11 in the second rear compressed groove 10c is preferably smaller than a pitch p_{b} of the high-compressed portions 11 in the first rear compressed groove 10b (p_{c} < p_{b}). This allows the rigidity of the second rear region C to be greater than the rigidity of the first rear region B. Therefore, the above-described effect to lower the rigidity of the first rear region B and to maintain the softness of the main body 8, enabling the main body to be well deformed following the shape of the intergluteal cleft, can be achieved, and the twists and the wrinkles in the second rear region C closer to the rear end can be prevented.

A pitch p_{c} of the high-compressed portions 11 in the second rear compressed groove 10c is preferably 3.5 to 7.5 mm, more preferably 4.5 to 6.5 mm. Further, the ratio of the p_{c} to the p_{b} (p_{c}/p_{b}) is preferably 0.5 to 0.9, more preferably 0.68 to 0.88. The pitch p_{c} of the high-compressed portions 11 in the second rear compressed groove 10c may be the same as the pitch pₐ of the high-compressed portions 11 in the middle compressed groove 10a.

Further, a distance d_{c} between the high-compressed portions 11 in the second rear compressed groove 10c is preferably smaller than the distance d_{b} between the high-compressed portions 11 in the first rear compressed groove 10b (d_{c} < d_{b}). The distance d_{c} between the high-compressed portions 11 in the second rear compressed groove 10c is preferably 2.5 to 5 mm, more preferably 3 to 4.5 mm.

The above-mentioned groove widths wₐ, w_{b}, and w_{c} each may be constant or vary along the compressed groove. Further, the pitches pₐ, p_{b}, and p_{c}, and the distances dₐ, d_{b}, and d_{c} each may also be constant or vary along the compressed groove. When the pitch or the distance varies along the compressed groove, the pₐ, p_{b}, p_{c}, dₐ, d_{b}, or d_{c} may be an averaged value in each compressed groove.

In the illustrated example, in the front-rear direction compressed grooves 10, 10, the high-compressed portions are formed to contact with one of the edges of the compressed groove. Specifically, a high-compressed portion contacted with the one edge of the compressed groove and a high-compressed portion contacted with the other edge of the compressed groove are alternately arranged to form a staggered arrangement. The staggered arrangement is preferred, because it allows the high-compressed portions to be formed with certainty in the fabrication. However, the arrangement of the high-compressed portions is not limited to the illustrated embodiment, and the high-compressed portions may be each formed to contact with both edges of the compressed groove and arranged at a predetermined distance.

Further, the shape of the high-compressed portion in the front-rear direction compressed groove 10, 10 is a half circle in FIG. 1, but may be a circle, an ellipse, a polygon such as a quadrangle. In any of the above cases, the high-compressed portions may be formed to contact with both edges of the compressed groove, or to contact with one of the edges of the compressed groove. For example, as in the later described front compressed groove 20, the high-compressed portion may be formed in a circular shape contacting with both edges of the compressed groove. Further, the high-compressed portions may be arranged contacting with neither edge of the compressed groove.

As shown in FIG. 1, the compressed groove may include the front compressed groove 20. With the configuration including the front compressed groove 20, the front region can be easily curved following the roundness of the human body, and prevent the leakage from the front side.

The shape of the front compressed groove 20 is also not particularly limited. However, a shape including a V-shape, such as a heart shape as in the illustrated example, is preferred, because the tip of the V-shape can be a base point for deforming the front region to be curved.

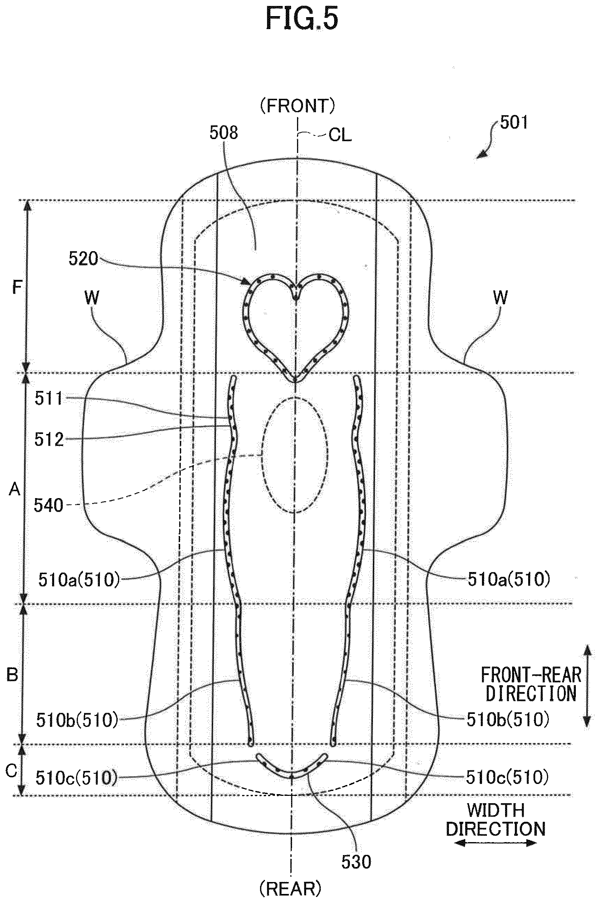

FIG. 5 shows an absorbent article 501 according to another example. Likewise in the example illustrated in FIG. 1, the absorbent article 501 includes a main body 508, and a compressed groove recessed from a topsheet 503 side toward a backsheet side. The compressed groove includes front-rear direction compressed grooves 510, 510, the front-rear direction compressed grooves 510, 510 including middle compressed grooves 510a, 510a and first rear compressed grooves 510b, 510b. However, the absorbent article 501 may differ from the absorbent article 1 shown in FIG. 1, in that the front-rear direction compressed grooves 510, 510 do not include second rear compressed grooves, but include a width direction rear compressed groove 530 in the second rear region C. Further, the length of the absorbent article 501 is shorter than the length of the absorbent article 1 by shortening the length of the second rear region C.

Also in the example shown in FIG. 5, in a plane view, a total area per unit length in the front-rear direction of high-compressed portions 511 in the first rear compressed grooves 510b, 510b is smaller than a total area per unit length in the front-rear direction of the high-compressed portions 511 in the middle compressed grooves 510a, 510a. Therefore, while maintaining the fittability in the middle region A, which corresponds to a crotch part of the wearer when attached, the rear first compressed region B, i.e., the intergluteal cleft corresponding region can be kept soft, thereby improving the fittability of the rear first compressed region B to the intergluteal cleft.

The width direction rear compressed groove 530 may also include high-compressed portions and a low-compressed portion. A width, a pitch of the high-compressed portions, or a distance between the high-compressed portions of the width direction rear compressed groove 530A may be the same as or different from those in the first rear compressed grooves 510b, 510b, and further be the same as or different from in the middle compressed grooves 510a, 510a.

### Reference Signs List

1, 501 absorbent article
2 backsheet
3, 503 topsheet
4 absorbent body
5 enveloping sheet
7 side nonwoven fabric
8, 508 main body (absorbent article main body)
10, 510 front-rear direction compressed groove
10a, 510a middle compressed groove
10b, 510b first rear compressed groove
10c second rear compressed groove
11, 511 high-compressed portion
12, 512 low-compressed portion
20 front compressed groove
40 body fluid discharge opening corresponding portion
70 intergluteal cleft
530 width direction rear compressed groove
CL front-rear direction centerline
A middle region
B first rear region (intergluteal cleft corresponding region)
C second rear region
F front region
W wing

## Claims

1. An absorbent article (1, 501) comprising a main body (8, 508) including a liquid-permeable topsheet (3, 503), a liquid-impermeable backsheet (2), and an absorbent body (4) disposed between the topsheet (3, 503) and the backsheet (2),
wherein the main body (8, 508) has an elongated shape with a predetermined length in the front-rear direction and a predetermined width in a width direction orthogonal to the front-rear direction, and includes a compressed groove recessed from a topsheet (3, 503) side toward a backsheet side,
wherein the compressed groove includes a pair of front-rear direction compressed grooves (10, 510) extending in the front-rear direction,
wherein the front-rear direction compressed grooves (10, 510) each include high-compressed portions (11, 511) and a low-compressed portion (12, 512) that is less deeply recessed than the high-compressed portions (11, 511), and each include a first rear compressed groove (10b, 510b) formed in a region corresponding to an intergluteal cleft (70) of a wearer when attached, and a middle compressed groove (10a, 510a) formed forward of the first rear compressed groove (10b, 510b), and
wherein a total area per unit length in the front-rear direction of the high-compressed portions (11, 511) in the first rear compressed groove (10b, 510b) is smaller than a total area per unit length in the front-rear direction of the high-compressed portions (11, 511) in the middle compressed groove (10a, 510a).

2. The absorbent article (1, 501) according to claim 1, wherein a groove width (Wb) of the first rear compressed groove (10b, 510b) is smaller than a groove width (Wa) of the middle compressed groove (10a, 510a).

3. The absorbent article (1, 501) according to claim 1, wherein a pitch (Pb) of the high-compressed portions (11, 511) in the first rear compressed groove (10b, 510b) is greater than a pitch (Pa) of the high-compressed portions (11, 511) in the middle compressed groove (10a, 510a).

4. The absorbent article (1) according to claim 1, wherein the front-rear direction compressed grooves (10) each include a second rear compressed groove (10c) formed backward of the first rear compressed groove (10b),
wherein a total area per unit length in the front-rear direction of the high-compressed portions (11) in the second rear compressed groove (10c) is greater than the total area per unit length in the front-rear direction of the high-compressed portions (11) in the first rear compressed groove (10b).

5. The absorbent article (1) according to claim 4, wherein a groove width (Wc) of the second rear compressed groove (10c) is greater than a groove width (Wb) of the first rear compressed groove (10b).

6. The absorbent article (1) according to claim 5, wherein a pitch (Pc) of the high-compressed portions (11) in the second rear compressed groove (10c) is smaller than the pitch (Pb) of the high-compressed portions (11) in the first rear compressed groove (10b).

## Patentansprüche

1. Absorbierender Artikel (1, 501) mit einem Hauptkörper (8, 508), der eine flüssigkeitsdurchlässige Oberschicht (3, 503), eine flüssigkeitsundurchlässige Unterschicht (2) und einen absorbierenden Körper (4) aufweist, der zwischen der Oberschicht (3, 503) und der Unterschicht (2) angeordnet ist,
wobei der Hauptkörper (8, 508) eine längliche Form mit einer vorbestimmten Länge in der Vorne-Hinten-Richtung und einer vorbestimmten Breite in einer Breitenrichtung orthogonal zu der Vorne-Hinten-Richtung aufweist und eine komprimierte Rille enthält, die von einer Seite der Oberschicht (3, 503) zu einer Seite der Unterschicht hin vertieft ist,
wobei die komprimierte Rille ein Paar von in der Vorne-Hinten-Richtung komprimierten Rillen (10, 510) aufweist, die sich in der Vorne-Hinten-Richtung erstrecken,
wobei die in Vorne-Hinten-Richtung komprimierten Rillen (10, 510) jeweils hoch-komprimierte Abschnitte (11, 511) und einen niedrig-komprimierten Abschnitt (12, 512) umfassen, der weniger tief vertieft ist als die hoch-komprimierten Abschnitte (11, 511), und jeweils eine erste hintere komprimierte Rille (10b, 510b), die in einem Bereich ausgebildet ist, der einer interglutealen Spalte (70) eines Trägers entspricht, wenn er befestigt ist, und eine mittlere komprimierte Rille (10a, 510a) umfassen, die vor der ersten hinteren komprimierten Rille (10b, 510b) ausgebildet ist, und
wobei eine Gesamtfläche pro Längeneinheit in der Vorne-Hinten-Richtung der hoch-komprimierten Abschnitte (11, 511) in der ersten hinteren komprimierten Rille (10b, 510b) kleiner ist als eine Gesamtfläche pro Längeneinheit in der Vorne-Hinten-Richtung der hoch-komprimierten Abschnitte (11, 511) in der mittleren komprimierten Rille (10a, 510a).

2. Absorbierender Artikel (1, 501) nach Anspruch 1, wobei eine Rillenbreite (Wb) der ersten hinteren komprimierten Rille (10b, 510b) kleiner ist als eine Rillenbreite (Wa) der mittleren komprimierten Rille (10a, 510a).

3. Absorbierender Artikel (1, 501) nach Anspruch 1, wobei ein Abstand (Pb) der hoch-komprimierten Abschnitte (11, 511) in der ersten hinteren komprimierten Rille (10b, 510b) größer ist als ein Abstand (Pa) der hoch-komprimierten Abschnitte (11, 511) in der mittleren komprimierten Rille (10a, 510a).

4. Absorbierender Artikel (1) nach Anspruch 1, wobei die in Vorne-Hinten-Richtung komprimierten Rillen (10) jeweils eine zweite hintere komprimierte Rille (10c) umfassen, die hinter der ersten hinteren komprimierten Rille (10b) ausgebildet ist,
wobei eine Gesamtfläche pro Längeneinheit in der Vorne-Hinten-Richtung der hoch-komprimierten Abschnitte (11) in der zweiten hinteren komprimierten Rille (10c) größer ist als die Gesamtfläche pro Längeneinheit in der Vorne-Hinten-Richtung der hoch-komprimierten Abschnitte (11) in der ersten hinteren komprimierten Rille (10b).

5. Absorbierender Artikel (1) nach Anspruch 4, wobei eine Rillenbreite (Wc) der zweiten hinteren komprimierten Rille (10c) größer ist als eine Rillenbreite (Wb) der ersten hinteren komprimierten Rille (10b).

6. Absorbierender Artikel (1) nach Anspruch 5, wobei ein Abstand (Pc) der hoch-komprimierten Abschnitte (11) in der zweiten rückseitigen komprimierten Rille (10c) kleiner ist als der Abstand (Pb) der hoch-komprimierten Abschnitte (11) in der ersten rückseitigen komprimierten Rille (10b).

## Revendications

1. Article absorbant (1, 501) comprenant un corps principal (8, 508) incluant une feuille supérieure (3, 503) perméable aux liquides, une feuille arrière (2) imperméable aux liquides, et un corps absorbant (4) disposé entre la feuille supérieure (3, 503) et la feuille arrière (2),
sachant que le corps principal (8, 508) a une forme allongée avec une longueur prédéterminée dans la direction avant-arrière et une largeur prédéterminée dans une direction de largeur perpendiculaire à la direction avant-arrière, et inclut une rainure comprimée évidée depuis un côté feuille supérieure (3, 503) vers un côté feuille arrière,
sachant que la rainure comprimée inclut une paire de rainures comprimées en direction avant-arrière (10, 510) s'étendant dans la direction avant-arrière,
sachant que les rainures comprimées dans la direction avant-arrière (10, 510) incluent chacune des parties hautement comprimées (11, 511) et une partie faiblement comprimée (12, 512) qui est moins profondément évidée que les parties hautement comprimées (11, 511), et incluent chacune une première rainure comprimée arrière (10b, 510b) formée dans une région correspondant à un sillon interfessier (70) d'un porteur lorsqu'il est attaché, et une rainure comprimée médiane (10a, 510a) formée en avant de la première rainure comprimée arrière (10b, 510b), et
sachant qu'une aire totale par unité de longueur dans la direction avant-arrière des parties hautement comprimées (11, 511) dans la première rainure comprimée arrière (10b, 510b) est plus petite qu'une aire totale par unité de longueur dans la direction avant-arrière des parties hautement comprimées (11, 511) dans la rainure comprimée médiane (10a, 510a).

2. L'article absorbant (1, 501) selon la revendication 1, sachant qu'une largeur de rainure (Wb) de la première rainure comprimée arrière (10b, 510b) est plus petite qu'une largeur de rainure (Wa) de la rainure comprimée médiane (10a, 510a).

3. L'article absorbant (1, 501) selon la revendication 1, sachant qu'un pas (Pb) des parties hautement comprimées (11, 511) dans la première rainure comprimée arrière (10b, 510b) est plus grand qu'un pas (Pa) des parties hautement comprimées (11, 511) dans la rainure comprimée médiane (10a, 510a).

4. L'article absorbant (1) selon la revendication 1, sachant que les rainures comprimées en direction avant-arrière (10) incluent chacune une deuxième rainure comprimée arrière (10c) formée en arrière de la première rainure comprimée arrière (10b),
sachant qu'une aire totale par unité de longueur dans la direction avant-arrière des parties hautement comprimées (11) dans la deuxième rainure comprimée arrière (10c) est plus grande que l'aire totale par unité de longueur dans la direction avant-arrière des parties hautement comprimées (11) dans la première rainure comprimée arrière (10b).

5. L'article absorbant (1) selon la revendication 4, sachant qu'une largeur de rainure (Wc) de la deuxième rainure comprimée arrière (10c) est plus grande qu'une largeur de rainure (Wb) de la première rainure comprimée arrière (10b).

6. L'article absorbant (1) selon la revendication 5, sachant qu'un pas (Pc) des parties hautement comprimées (11) dans la deuxième rainure comprimée arrière (10c) est plus petit que le pas (Pb) des parties hautement comprimées (11) dans la première rainure comprimée arrière (10b).
